# Europäisches Patentamt
# European Patent Office
# Office européen des brevets

(19)

(11) Publication number: **0 036 902**
**B1**

(12) **EUROPEAN PATENT SPECIFICATION**

(45) Date of publication of patent specification: **27.04.83**

(51) Int. Cl.³: **A 23 K 1/16, A 61 K 9/14**

(21) Application number: **80101745.0**

(22) Date of filing: **01.04.80**

(54) **Process for the production of water-soluble biotin-containing preparations and their use as additives to feed supplements.**

(43) Date of publication of application:
**07.10.81 Bulletin 81/40**

(45) Publication of the grant of the patent:
**27.04.83 Bulletin 83/17**

(84) Designated Contracting States:
**BE DE FR GB IT**

(56) References cited:
**DE - A - 2 751 614**
**DE - A - 2 822 324**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Mitsunaga, Takayoshi**
**6-4-23, Yamate-dai**
**Ibaraki Osaka (JP)**
Inventor: **Chinushi, Kiyoto**
**No. 1-206, 1-26, Tamagawa**
**Takatsuki Osaka (JP)**
Inventor: **Umezu, Tadashi**
**9-611, Ogawa-cho**
**Ibaraki Osaka (JP)**

(74) Representative: **Vossius Vossius Tauchner Heunemann Rauh**
**Siebertstrasse 4 P.O. Box 86 07 67**
**D-8000 München 86 (DE)**

Courier Press, Leamington Spa, England.

# Process for the production of water-soluble biotin-containing preparations and their use as additives to feed supplements

The present invention relates to a water-soluble biotin-containing preparation and its production.

As well known, biotin is used as an additive to feed supplements for domestic animals and culture fishes. It is also used as a medicament for the prevention and treatment of skin diseases.

Since, however, biotin itself is hardly soluble in water, various inconveniences are encountered on its practical use. For instance, a method for mixing the active ingredient uniformly into feed supplements by dissolving the active ingredient in water and spraying the resultant solution onto the feed supplements is not applicable. Further, for instance, a method for administering the active ingredient orally to domestic animals by dissolving the active ingredient in drinking water or liquid feed supplements and giving the resulting solution to the domestic animals is not applicable. In addition, since its absorption rate in domestic animals is low, it is slow to reveal any practical effect.

In order to overcome said drawbacks, extensive research has been undertaken with the aim of increasing the solubility of biotin in water. As a result, it has now been found that dried particles containing biotin obtained by spray-drying an aqueous solution comprising biotin and lactose with ammonia is highly soluble in water. This finding is unexpected, because a mixture of biotin and lactose obtained merely by mechanical blending does not materially contribute to increasing the water-solubility of biotin. It is also notable that dried particles containing biotin obtained by spray-drying an aqueous solution comprising biotin and ammonia without lactose do not show good water-solubility.

The water-soluble biotin-containing preparation of the present invention can be prepared by spray-drying an aqueous solution comprising biotin, lactose and ammonia to obtain dried particles containing biotin.

The aqueous solution to be subjected to spray-drying should contain biotin, lactose and ammonia. The weight proportion of biotin and lactose in the aqueous solution is usually from 1:40 to 1:100. It is desirable for the biotin to be completely dissolved in the aqueous solution. In order to achieve such complete dissolution, the presence of ammonia is required. The amount of ammonia in the aqueous solution should be sufficient to dissolve the biotin in the aqueous solution. When, for instance, 28% aqueous ammonia is used, it may be usually employed to make a concentration of 0.1% (w/v) or more in the aqueous solution. The resulting aqueous solution ordinarily has a pH of 5 to 9.

There are no special conditions limiting the procedure for preparing the aqueous solution.

For instance, the aqueous solution may be prepared by adding lactose to water containing ammonia and then adding biotin thereto. Alternatively, for instance, it may be prepared by adding a mixture of lactose and biotin to water containing ammonia. Furthermore, for instance, it may be prepared by dispersing lactose and biotin in water and then adding ammonia thereto.

If desired, the aqueous solution may contain any additive such as a carrier (e.g. glucose, fructose, sucrose), a preservative (e.g. sorbic acid, p-hydroxybenzoic esters) or a pigment in addition to the said essential components.

The thus prepared aqueous solution is subjected to spray-drying. The spray-drying may be carried out by a conventional procedure. As a result dried particles were obtained, each particle of which comprises lactose and biotin uniformly dispersed therein.

The dried particles are usually spherical and have good flow properties. Accordingly, as such they can be evenly and easily mixed into feed supplements. Since the dried particles are low in hygroscopicity, they hardly form blocks even in the atmosphere. It is particularly characteristic that the dried particles are highly soluble in water. Therefore, they can be given to domestic animals by dissolving in drinking water or liquid feed supplements. This is a quite convenient feature of the practical use of biotin.

Practical and presently preferred embodiments of the invention are illustrated in the following Examples.

Example 1

Biotin (10 g) and lactose (490 g) were added to water (2000 ml) containing 28% aqueous ammonia in a concentration of 0.25% (w/v), followed by heating at a temperature of 60 to 70°C. The resulting solution was spray-dried with the aid of a spray drier under the following conditions to give spherical particles of 5 to 40 microns in particle size: chamber temperature, 130 to 140°C; chamber vacuum, 30 mm $H_2O$; atomizing pressure, 2.5 kg/cm$^2$.

The thus obtained particles have good flow properties and a critical specific humidity of 92%. Thus, they are low in hygroscopicity.

Comparative Example 1

Biotin (10 g) and lactose (490 g) were mixed together with the aid of a V-shaped blender for 30 minutes to give a biotin preparation containing biotin in a concentration of 2% (w/w).

Comparative Example 2

Biotin (10 g) was added to water (2000 ml) containing 28% aqueous ammonia in a concentration of 0.25% (w/v), followed by heating at a temperature of 60 to 70°C. The resulting solution was spray-dried in the same manner as in Example 1 to give particles.

Comparative Example 3

Biotin (10 g) and lactose (490 g) were added to water (2000 ml), followed by heating at a temperature of 60 to 70°C while stirring. The resulting dispersion was spray-dried in the same manner as in Example 1 to give particles.

The products in Example 1 and Comparative Examples 1 to 3 were each dissolved in water at room temperature, and the water-solubility of biotin in each product was determined by quantitative measurement of the biotin using high-speed liquid chromatography. The results are shown in Table 1.

TABLE 1

| Product | Amount of biotin (mg) dissolved in 100 ml of water |
|---|---|
| Example 1 | 400 |
| Comparative Example 1 | 30 |
| Comparative Example 2 | 40 |
| Comparative Example 3 | 30 |

From the above results, it can be seen that the spray-drying of an aqueous solution comprising biotin and lactose with ammonia is quite effective in increasing the water-solubility of biotin.

Example 2

Biotin (10 g) was added to water (2000 ml) containing 28% aqueous ammonia in a concentration of 0.5% (w/v), and lactose (490 g) was added thereto, followed by heating at a temperature of 60 to 70°C. The resulting solution was spray-dried in the same manner as in Example 1 to give spherical particles of 5 to 40 microns in particle size.

The thus obtained particles have a critical specific humidity of 91%. Thus, they are low in hygroscopicity. Further, the amount of biotin in them dissolved in 100 ml of water at room temperature was 400 mg.

Example 3

Biotin (10 g) and lactose (990 g) were added to water (10 liters) containing 28% aqueous ammonia in a concentration of 0.1% (w/v), followed by heating at a temperature of 60 to 70°C. The resulting solution was spray-dried in the same manner as in Example 1 to give spherical particles of 5 to 40 microns in particle size.

The thus obtained particles have a critical specific humidity of 94%. Thus, they are low in hygroscopicity. Further, the amount of biotin in them dissolved in 100 ml of water at room temperature was 200 mg.

Example 4

Biotin (10 g) and lactose (490 g) were mixed together and dispersed in water (2000 ml). Then, 28% aqueous ammonia (10 ml) was added thereto, followed by heating at a temperature of 60 to 70°C. The resulting solution was spray-dried in the same manner as in Example 1 to give spherical particles of 5 to 40 microns in particle size.

The thus obtained particles have a critical specific humidity of 92%. Thus, they are low in hygroscopicity. Further, the amount of biotin in them dissolved in 100 ml of water at room temperature was 350 mg.

Example 5

Biotin (10 g), glucose (50 g), sorbic acid (1 g) and lactose (939 g) were added to water (10 liters) containing 28% aqueous ammonia in a concentration of 0.15% (w/v), followed by heating at a temperature of 60 to 70°C. The resulting solution was spray-dried in the same manner as in Example 1 to give spherical particles of 5 to 40 microns in particle size.

The thus obtained particles have a critical specific humidity of 88%. Thus, they are low in hygroscopicity. Further, the amount of biotin in them dissolved in 100 ml of water at room temperature was 260 mg.

**Claims**

1. A process for preparing a water-soluble biotin-containing preparation which comprises spray-drying an aqueous solution comprising biotin, lactose and ammonia to obtain dried particles containing biotin.

2. The process according to claim 1, wherein the weight proportion of biotin and lactose in the aqueous solution is from 1:40 to 1:100.

3. The process according to claim 1, wherein the aqueous solution was a pH of 5 to 9.

4. The process according to claim 1, wherein the aqueous solution further comprises a carrier and a preservative.

5. The process according to claim 4, wherein the carrier is glucose, fructose or sucrose.

6. The process according to claim 4, wherein the preservative is sorbic acid or a p-hydroxybenzoic ester.

7. Use of the preparations produced according to claim 1 as additives to feed supplements.

**Patentansprüche**

1. Ein Verfahren zur Herstellung einer wasserlöslichen, Biotin enthaltenden Zubereitung, das Sprühtrocknen einer Biotin, Laktose und Ammoniak enthaltenden wäßrigen Lösung einschließt, wobei getrocknete Biotin enthaltende Partikel erhalten werden.

2. Das Verfahren nach Anspruch 1, wobei das Gewichtsverhältnis von Biotin zu Laktose in der wäßrigen Lösung 1:40 bis 1:100 beträgt.

3. Das Verfahren nach Anspruch 1, wobei die wäßrige Lösung einen pH-Wert von 5 bis 9 hat.

4. Das Verfahren nach Anspruch 1, wobei die wäßrige Lösung zusätlich einen Trägerstoff und ein Konservierungsmittel enthält.

5. Das Verfahren nach Anspruch 4, wobei der Trägerstoff Glukose, Fructose oder Rohrzucker ist.

6. Das Verfahren nach Anspruch 4, wobei das Konservierungsmittel Sorbinsäure oder ein p-Hydroxybenzoesäureester ist.

7. Verwendung der Zubereitungen hergestellt nach Anspruch 1 als Zusatz zu Futtermittelzusatzstoffen.

**Revendications**

1. Procédé de formation d'une préparation contenant de la biotine soluble dans l'eau qui comprend le séchage par pulvérisation d'une solution aqueuse contenant de la biotine, du lactose et de l'ammoniaque de manière à obtenir des particules séchées contenant de la biotine.

2. Procédé selon la revendication 1, où la proportion en poids de biotine et de lactose dans la solution aqueuse est comprise entre 1:40 et 1:100.

3. Procédé selon la revendication 1, où la solution aqueuse a un pH comprise entre 5 et 9.

4. Procédé selon la revendication 1, où la solution aqueuse comprend en outre un agent de support et un agent de conservation.

5. Procédé selon la revendication 4 où l'agent de support est le glucose, le fructose ou le saccharose.

6. Procédé selon la revendication 4 où l'agent de conservation est l'acide sorbique ou un ester p-hydroxybenzoïque.

7. Utilisation des préparations produites selon la revendication 1, comme additifs à des compléments d'alimentation.